# EUROPÄISCHE PATENTANMELDUNG

(11) **EP 2 471 548 A1**
(43) Veröffentlichungstag der Anmeldung: **04.07.2012**
(21) Anmeldenummer: 10197146.3
(22) Anmeldetag: 28.12.2010
(51) Int. Cl.: A61K 38/20, C12N 15/113, A61P 35/00

(54) **siRNA gegen Cbl-b und optional IL2 und IL12 zur Verwendung in der Behandlung von Krebs**

(71) Anmelder: Apeiron Biologics AG, 1030 Wien (AT)
(72) Erfinder: Lametschwandtner, Günther, 1030 Wien (AT); Loibner, Hans, 1230 Wien (AT); Schuster, Manfred, 2191 Schrick (AT); Haslinger, Isabella, 1030 Wien (AT); Seidl, Sandra, 1230 Wien (AT)
(74) Vertreter: Sonn & Partner Patentanwälte

(57) **Zusammenfassung**

Die vorliegende Erfindung betrifft ein Verfahren zur Immunaktivierung von NK-Zellen durch Reduzierung oder Inhibierung der Cbl-b Funktion in den NK-Zellen. Dadurch wird das angeborene Immunsystem stimuliert wodurch die Therapie geeigneter Krankheiten ermöglicht wird.

SEQUENCE LISTING

<110>  APEIRON BIOLOGICS AG

<120>  Verfahren zur Aktivierung von NK-Zellen

<130>  r57553

<160>  4

<170>  PatentIn version 3.4


<210>  1
<211>  21
<212>  RNA
<213>  Artificial

<220>
<223>  RNAi

<400>  1
guacuggucc guuagcaaau u                                                21


<210>  2
<211>  21
<212>  RNA
<213>  Artificial

<220>
<223>  RNAi

<400>  2
uuugcuaacg gaccaguacu u                                                21


<210>  3
<211>  21
<212>  RNA
<213>  Artificial

<220>
<223>  RNAi

<400>  3
ggucgaauuu uggguauuau u                                                21


<210>  4
<211>  21
<212>  RNA
<213>  Artificial

<220>
<223>  RNAi

<400>  4
uaauacccaa aauucgaccu u                                                21

## Beschreibung

Die vorliegende Erfindung betrifft therapeutische Verfahren zur Aktivierung des angeborenen Immunsystems, insbesondere von NK-Zellen.

NK-Zellen (natürliche Killerzellen) gehören zu den Lymphozyten (Untergruppe der weißen Blutzellen oder Leukozyten). Sie sind in der Lage abnormale Zellen, wie Tumorzellen und virusinfizierte Zellen, zu erkennen und abzutöten. NK-Zellen besitzen keine Antigen-spezifischen Rezeptoren und gehören zum angeborenen Immunsystem. NK-Zellen erkennen unter anderem den MHC-I-Komplex, der auf nahezu allen gesunden Körperzellen vorkommt. Wird eine Zelle durch Viren infiziert oder wandelt sie sich in eine Tumorzelle um, so geht unter Umständen der MHC-I-Komplex auf der Oberfläche verloren. NK-Zellen entwickeln sich wie die anderen Lymphozyten aus lymphatischen Vorläuferzellen im Knochenmark und zirkulieren später im Blutkreislauf.

Loeser et al. (JEM (2007) doi:10.1084/iem.20061699) zeigten, dass Cbl-b ein negativer Regulator ist, der maßgeblich für die "Immunreaktivität" von T-Zellen verantwortlich ist. Cbl-b unterdrückt die Aktivierung von T-Zellen und ist in der Lage Autoimmunreaktion zu verhindern. In Abwesenheit von Cbl-b können verabreichte aber kaum immunogene Substanzen zur Induktion einer starken Immunantwort führen. Ferner sind Cbl-b defiziente Mäuse (homozygotischer Gen knock-out) lebensfähig und deren Immunsystem ist in der Lage effizient autolog-induzierte Tumore zu erkennen und dagegen eine vor allem auf CD8+ T-Zellen-beruhende lytische Immunantwort aufzubauen. Allerdings führte die beschriebene, gänzliche Abschaltung des Enzyms ebenfalls zu einer erhöhten Autoimmunität nach Immunisierung mit Superantigenen.

Chiang et al. (Journal of Clinical Investigation 117 (4) (2007): 1033-1034) schreiben, dass Cbl-b-/- CD8⁺ T-Zellen verwendet werden können, um die anti-Tumor-Reaktivität in E.G7-Mäusen zu erhöhen.

Die WO 2008/033403 A beschreibt die Erhöhung der Reaktivität von CD8+ T-Zellen durch Reduzierung der Cbl-b Aktivität. Inhibitorische RNA Sequenzen, insbesondere von siRNA, gegen Cbl-b wird offenbart.

Lametschwandtner et al. (Journal of Immunotherapy 31 (9) (2008): 943) beschreiben Immuntherapien, welche auf der Unterdrückung von Cbl-b in T-Zellen beruhen.

Somit war bekannt, dass T-Zellen, also Zellen des adaptiven Immunsystems, mittels Cbl-b Inhibition aktiviert werden können um eine Immunantwort zu fördern. Eine solche Förderung der Immunantwort ist besonders für schwere chronische Krankheiten von therapeutischem Interesse, bei denen die nicht ausreichende Aktivität des Immunsystems kausal für den Verlauf der Erkrankung ist. Solche Erkrankungen sind beispielsweise chronische Infektionen oder Tumorerkrankungen. Allerdings bedürfen gerade effektive Immunantworten in diesen Erkrankungen eines effizienten Zusammenspiels des adaptiven und des angeborenen Immunsystems, wobei insbesondere zur Aktivierung von NK-Zellen noch keine ausreichenden Behandlungsansätze klinisch verfügbar sind.

Es ist daher ein vorrangiges Ziel neue Verfahren zu finden, welche durch die Aktivierung des angeborenen Immunsystems unter besonderer Bezugnahme auf NK-Zellen somit zu einer stark verbesserten Effektivität von Immunantworten führen können.

Erfindungsgemäß wurde dieses Ziel durch die Inhibition von Cbl-b in NK-Zellen gelöst.

Die Erfindung betrifft in einem ersten Aspekt ein Verfahren zur Immunaktivierung von NK-Zellen umfassend den Schritt der Reduzierung oder Inhibierung der Cbl-b Funktion in den NK-Zellen. Die Inhibierung kann durch Verabreichung eines Cbl-b Inhibitors erzielt werden. Ein solcher Inhibitior kann direkt in vivo an einen Patienten verabreicht werden.

In einem gleichwertigen damit verbundenen Aspekt betrifft die Erfindung einen Cbl-b Inhibitor zur Verwendung in einem Verfahren zur therapeutischen Behandlung eines Patienten umfassend die Einbringung des Cbl-b Inhibitors in NK-Zellen im Patienten. Durch diese Behandlung können die NK-Zellen aktiviert werden.

"Verabreichung eines Cbl-b Inhibitors" oder "Cbl-b Inhibierung" sind Begriffe die hierin, insbesondere bei der Beschreibung spezieller Ausführungsformen, austauschbar verwendet werden.

Die erfindungsgemäße Inhibition von Cbl-b kann für Immuntherapien in einem Patienten verwendet werden, insbesondere zur Aktivierung des Immunsystems bzw. des angeborenen Immunsystems, speziell vermittelt durch NK-Zellen. Insbesondere kann das erfindungsgemäße Verfahren zur Behandlung von Krebs, einer Virusinfektion, einer bakteriellen Infektion, insbesondere einer chronischen Infektion, im Besonderen einer chronische Infektion mit intrazellulär persistenten Bakterien, oder einer Parasiteninfektion, insbesondere einer Mykose, in dem Patienten eingesetzt werden. Die erfindungsgemäße NK-Zell-Aktivierung mittels Cbl-b Inhibierung ist bei derartigen Krankheiten besonders effektiv. Der Patient ist vorzugsweise ein Säugetier, im speziellen ein Mensch.

Insbesondere im Fall von Krebs lässt sich die erfindungsgemäße NK-Zell Behandlung mit herkömmlichen Therapien kombinieren. Viele Tumortherapien, wie zB. Strahlungstherapie, Chemotherapie oder operative Tumorentfernungen, sind seit Jahren etabliert und werden ständig verfeinert und verbessert. Neue Therapien umfassen Immuntherapien und Therapien, die auf spezifische Marker von Tumorzellen abzielen, insbesondere unter Verwendung von monoklonalen Antikörpern. Gerade die Wirkung letzterer hängt auch wesentlich von der Aktivität der NK-Zellen ab, die die tumorzellgebundenen Antikörper über generelle Antikörper-Determinanten erkennen und in weiterer Folge die Tumorzelle abtöten. Durch die Aktivierung des angeborenen Immunsystems über die Wirkung der NK-Zellen steht somit eine weitere Strategie zur Verfügung, die bisherige Ansätze ergänzen und vervollständigen kann um möglichst breit Immunreaktionen zu fördern, im Speziellen zur Bekämpfung von Krebszellen. Insbesondere Therapien, welche einen direkten zellschädigenden Einfluss auf die Tumorzellen haben, z.B. Chemotherapien oder Strahlentherapien, induzieren die Expression von Molekülen der MHC-Klasse und anderen immunaktivierenden Rezeptoren, bspw. von NKG2D-Liganden. Diese zellulären Veränderungen werden von Zellen des angeborenen Immunsystems, insbesondere NK-Zellen, erkannt und führen zu deren Aktivierung, welche durch die Synergie mit der erfindungsgemäßen NK-Zell-Aktivierung eine wesentlich höhere therapeutische Wirkung erzielen kann.

Demgemäß ist die erfindungsgemäß zu behandelnde Krebserkrankung vorzugsweise ausgewählt aus Krebserkrankungen des reproduktiven Traktes, insbesondere Eierstockkrebs, Hodenkrebs, Prostatakrebs oder Brustkrebs; Krebserkrankungen des Verdauungstraktes, insbesondere Magenkrebs, Darmkrebs, Rektalkarzinom, Pankreaskrebs, Speiseröhrenkrebs und Leberkrebs; Nierenkrebs, Hautkrebs, insbesondere Melanomen, Basalzellkarzinomen und Plattenepithelkarzinomen; Neuroblastomen und Glioblastomen, Lungenkrebs, Schilddrüsenkrebs, Sarkomen, Kopf-/Halskarzinom, Plattenepithelkarzinomen, Lymphomen und Leukämien (wobei hierin die Ausdrücke "Krebs,", "Tumor", "Karzinom", etc. immer synonym verwendet werden und sich auf bösartige Erkrankungen beziehen.

Das Cbl-b Gen sowie dessen Genprodukte sind im Stand der Technik ausführlich beschrieben (UniGene Id. Hs.3144 und Hs.381921). Cbl-b Sequenzen sind z.B. in der GenBank Datenbank unter den Acc. Nr. NM_008279 und NP_009112 veröffentlicht. Anti-Cbl-b Antikörper, siRNAs und Antisense-Inhibitoren sind kommerziell erhältlich. Bestimmte siRNAs geeignet zur Reduzierung oder Inhibierung der Cbl-b Expression und damit auch der Cbl-b Funktion sind beispielsweise in der US 2007/0054355 mit gemischten RNA/DNA Nukleotiden und einer Länge von ca. 20 Basen offenbart.

Cbl-b Inhibitoren sind im Stand der Technik hinlänglich bekannt. Beliebige Cbl-b Inhibitoren können gemäß der vorliegenden Erfindung eingesetzt werden. Vorzugsweise ist der Cbl-b Inhibitor ausgewählt aus inhibierenden Nukleinsäuren, insbesondere antisense Oligonukleotiden, insbesondere antisense RNA, siRNA (small inerfering RNA) oder shRNA (short haipin RNA). Nukleinsäure Inhibitoren können als solche verwendet werden oder in Form von Vektoren, welche die Inhibierung kodieren und exprimieren. Geeignete Cbl-b Inhibitoren sind z.B. Antagonisten, Aptameren oder Intrameren, vorzugsweise wird Cbl-b siRNA eingesetzt. siRNA Technologie zur Attenuierung der spezifischen Gen-Expression ist für Cbl-b bereits beschrieben. Die US 2007/0087988 betrifft ein Verfahren zur Regulierung der HPK1, dessen Expression durch die Erhöhung der Cbl-b Expression erhöht werden kann und vice versa (z.B. durch Cbl-b siRNA Inhibition).

Vorzugsweise wird die Cbl-b Funktion durch Reduzierung oder Inhibierung der Expression von Cbl-b reduziert oder inhibiert. Die Begriffe Reduzierung oder Inhibierung betreffen die Absenkung der Funktion (bzw. der Expression) von Cbl-b im Vergleich zu unveränderter, natürlicher Funktion bis zur kompletten Inhibierung der Funktion. Vorzugsweise wird die Funktion (bzw. Expression) um mindestens 30%, 40%, 50%, 60%, 70%, 80%, 90% oder 95% reduziert.

Die Reduzierung oder Inhibierung der Funktion von Cbl-b erfolgt in vorzugsweisen Ausführungsformen transient. D.h. die Funktion wird nur vorläufig w.o. angegeben reduziert und kann sich in Folge dessen wieder erholen, z.B. durch Verbrauch oder Abbau von Inhibitoren, wie z.B. Cbl-b siRNA, oder durch Neubildung oder nicht Cbl-b beeinträchtigter Zellen in vivo. Die transiente Reduzierung von Cbl-b in Immunzellen kann dabei auch repetitiv erfolgen, z.B. bis ein therapeutischer Erfolg erzielt wurde.

Vorzugsweise wird die Expression von Cbl-b durch Verwendung von Cbl-b antisense RNA oder siRNA reduziert oder inhibiert. Hierzu werden kurze DNA und/oder RNA Sequenzen komplementär zu einem der Teile der Ziel (Cbl-b) mRNA Sequenz eingesetzt, so dass diese damit hybridisieren und inaktivieren. Die Länge dieser Sequenzen ist vorzugsweise mindestens 15, 18, 20, 22, 25, 28, 30, 35, 40, 50, 60, 70, 80, 90, 100, 120, 140, 160, 180 oder 200 Basen bis zur kompletten Ziel Sequenz, vorzugsweise bis zu 2500, 2000, 1500, 1000, 500 oder 300 Basen. Vorzugsweise werden die Sequenzen der SEQ ID Nr. 1, 2, 3, 4, 5, 6, 7 und/oder 8 verwendet.

Ebenfalls kann die Funktion von Cbl-b durch eine Vielzahl weiterer bekannter Komponenten reduziert oder inhibiert werden, wie beispielsweise durch Verwendung von Cbl-b Antagonisten, Inhibitoren, insbesondere Aptameren oder Intrameren. Jegliche Antagonisten oder Inhibitoren, die die Wirkung bzw. die Funktion von Cbl-b unterdrücken können erfindungsgemäß verwendet werden um die Immunreaktivität der NK-Zellen zu erhöhen. Zur Inhibition von Cbl-b können auch Substanzen verwendet werden, die entweder spezifisch die enzymatische E3-Ligase-Aktivität inhibieren oder die intrazelluläre Assoziation von Cbl-b mit seinen Interaktionspartner inhibieren oder die die Expression von Cbl-b inhibieren. Vorzugsweise werden Antagonisten oder Inhibitoren zur Herstellung eines pharmazeutischen Mittels zur erfindungsgemäßen Erhöhung der Immunreaktivität der NK-Zellen verwendet. Ermöglicht werden Behandlungen von Krankheiten mit supprimiertem oder ineffizientem Immunsystem, insbesondere Krebs oder chronische Infektionen.

Erfindungsgemäß wurde festgestellt, dass die Inhibierung von Cbl-b zusammen mit anderen NK-Zell-stimulierenden Substanzen (NK-Zell-Aktivatoren) einen synergistischen Effekt bewirkt, der über die zu erwartenden Effekte der additiven Effekte der Inhibition von Cbl-b und NK-Zell-Aktivierung hinausgeht. Der Verabreichung des Cbl-b Inhibitors bzw. die Cbl-b Inhibition erfolgt daher vorzugsweise zusammen mit einer weiteren NK-Zell stimulierenden Substanz. Eine solche NK-Zell-stimulierende Substanz ist eine andere Substanz als der erfindungsgemäße Cbl-b Inhibitor. Vorzugsweise bewirkt die NK-Zell-stimulierende Substanz die Produktion von IFN-gamma durch die NK-Zellen, unabhängig von einer Cbl-b Inhibierung.

In einer speziellen Ausführungsform betrifft die vorliegende Erfindung die gemeinsame Verabreichung des Cbl-b Inhibitors mit einem NK-Zell-Aktivator, insbesondere ausgewählt aus einem Immunzell-stimmulierenden Zytokin bspw. aus einem Zytokin der common-gamma-chain Zytokine, insbesondere IL-2, IL-15, IL-21; Zytokine die sowohl Zellen des adaptiven als auch angeborenen Immunsystems stimulieren, insbesondere IL-12, IL-23, IL-27; Effektor-Zellzytokinen wie IL-1, IL-17, IL-18, einem Interferon oder Interferon-Stimulans, insbesondere wobei das Interferon-alpha einen Antikörper, insbesondere einen Antikörper der Tumorzelloberflächenmoleküle erkennt und/oder einen Antikörper dessen konstante Region an den entsprechenden Fc-Rezeptor an NK-Zellen binden kann, oder einem TLR- oder PAMP-Rezeptor Liganden, insbesondere Agonisten, vorzugsweise von TLR-1, TLR-2, TLR-3, TLR-7, TLR-8 oder TLR-9 sowie Kombinationen hievon. Insbesondere bevorzugt ist die Verabreichung des Cbl-b Inhibitors zusammen mit mindestens einem aus IL-2, IL-15, IL-12, IL-23, Interferon, einem Interferon-Stimulans, Imiquimod und andere TLR7/8-Agonisten, wie z.B. Resiquimod, ssPolyU-Nukleotide, Loxoribine, Gardiquimod, CL075, CL097, CL264, 3M002; poly(I:C) Oligonukleotide, CpG Oligonukleotide, CD205-Liganden oder CD206-Liganden, sowie Kombinationen hievon. Mögliche Kombinationen umfassen z.B. ein Zytokin der common-gamma-chain Zytokine mit einem der obigen NK-Zell-Aktivatoren bzw. ein Zytokin des adaptiven als auch angeborenen Immunsystems mit einem der obigen NK-Zell-Aktivatoren. Besonders bevorzugte Kombinationen sind mit einem Zytokin der common-gamma-chain Zytokine und einem Zytokin des adaptiven als auch angeborenen Immunsystems, insbesondere IL-2 und IL-12.

Vorzugsweise bewirken die eingesetzten NK-Zell-stimulierenden Substanzen eine IFN-gamma Produktion in NK-Zellen. IFN-alpha, IL-12 oder IL-23 bewirken beispielsweise besonders starke IFN-gamma-Antworten in NK-Zellen. Überraschend wurde festgestellt, dass die Aktivierung von NK-Zellen durch Inhibierung von Cbl-b mit IFN-gamma-Produktion-verursachenden NK-Zell-stimulierenden Substanzen besonders starke synergistische Wirkungen hervorrufen, die weit über die zu erwartende Wirkung der Einzelsubstanzen hinausgeht.

Ebenso kann der Cbl-b Inhibitor zusammen mit einem Antikörper gegen Tumorzelldeterminanten verabreicht werden, und optional zusätzlich auch kombiniert mit einem der oben angeführten Immunaktivatoren bzw. NK-Zell-stimulierenden Substanzen. Die Inhibition von Cbl-b in NK-Zellen als auch die Verabreichung des zusätzlichen NK-Zell-Aktivators bzw. des gegen Tumorzellen gerichteten Antikörpers können in vivo z.B. durch direkte Verabreichung an den Patienten erfolgen.

Vorzugsweise ist der Cbl-b Inhibitor an einen Liganden eines NK-Zell-Erkennungsmoleküls, z.B. eines NK-Zell-Oberflächenmoleküls, gekoppelt. Ein solcher Ligand kann bspw. ein natürlich vorkommendes Protein oder anderes Biomolekül sein oder ein funktionelles Derivat davon, welches NK-Zellen binden kann. Insbesondere kann ein solcher Ligand ein Antikörper gegen ein NK-Zellerkennungsmolekül sein. Erfindungsgemäß werden vorzugsweise spezifisch die NK-Zellen durch Cbl-b-Inhibierung aktiviert, z.B. durch Kopplung an einem solchen NK-Zell-Erkennungsmolekül, in vivo. Die "spezifische" NK-Zell Aktivierung soll als erhöhte Wirkung auf NK-Zellen verstanden werden im Vergleich zu unspezifischen, z.B. ungesteuerten bzw. ungekopppelten Cbl-b-Inhibitor Verabreichungen, welche auch in anderen Zellen Wirkung entfalten können. Eine unspezifische Verabreichung erfolgt z.B. durch einfache Verabreichung des Inhibitors ohne zusätzliche Modifikationen durch Kopplung an ein NK-Zell-Erkennungsmolekül. Durch die spezifische NK-Zell Aktivierung lässt sich gezielt die erfindungsgemäße NK-Zell-vermittelte Immunantwort (angeborenes Immunsystem) steuern, mit geringeren oder ungewünschten Nebenwirkungen, z.B. durch das adaptive Immunsystem.

Unter dem Begriff "Antikörper" werden alle natürlich vorkommenden Antikörper verstanden, wie IgG-, IgD-, IgA-, IgE-, IgM-Antikörper, sowie deren funktionelle Derivate, welche beispielsweise Fab-, Fab'-, F(ab)₂-, F(ab')₂-Fragmente, einzelkettige Antikörper (scAb oder scFv), oder ein Fragment einer variablen Antikörperdomäne, umfassen und ein Antigen, hier insbesondere ein NK-Zell-Erkennungsmolekül, spezifisch binden bzw. dagegen gerichtet sind. Der Antikörper kann monoklonal oder polyklonal sein. Antikörper, welche wie zuvor beschrieben in einer Therapie in Kombination mit dem erfindungsgemäßen Cbl-b-Inhibitor verabreicht werden sollen, sind gegen ein Epitop eines Krankheitserregers gerichtet, oder ein Tumorepitop, und weisen vorzugsweise eine Fc-Domäne auf.

Zu den bevorzugten NK-Zellerkennungsmolekülen gehören insbesondere solche die entweder spezifisch nur auf NK-Zellen vorkommen oder die besonders häufig auf NK-Zellen exprimiert sind und gegebenenfalls zusätzlich noch auf weiteren Immunzellen deren Aktivität wunschgemäß durch Cbl-b Inhibierung erhöht werden kann bspw. CD2, CD8a, CD16, CD25, CD27, CD56, CD71, CD158, CD159, CD160, CD161, CD205, CD206, CD205, CD314, CD335, CD336, CD337. Dadurch kann der Cbl-b Inhibitor entweder spezifisch an NK-Zellen oder an NK-Zellen und relevante weitere Subtypen von Immunzellen in einem Patienten gezielt herangebracht und durch die Zellen aufgenommen werden. Damit kann bewirkt werden, dass nur spezifisch in den gewünschten Ziel-Zellen der Cbl-b Inhibitor therapeutisch wirksam wird.

In einem weiteren Aspekt betrifft die vorliegende Erfindung eine pharmazeutische Zusammensetzung umfassend einen Cbl-b Inhibitor und einen zusätzlichen NK-Zell Aktivator wie oben beschrieben, insbesondere Immunzell-stimmulierendem Zytokin, einem Interferon oder Interferon-Stimulans, einem Antikörper, oder einem TLR- oder PAMP-Rezeptor Liganden. Eine solche Zusammensetzung kann für die oben genannten Zwecke zur Inhibition von Cbl-b alleine oder in Kombination mit weiteren NK-Zell Aktivatoren eingesetzt werden.

Vorzugsweise umfasst die Zusammensetzung einen pharmazeutisch akzeptablen Träger, vorzugsweise geeignet zur intrazellulären Verabreichung in einem Patienten, insbesondere Vehikel wie Liposomen oder Mikrosomen Formulierungen, welche insbesondere zur Verabreichung von Nukleinsäuren bevorzugt werden. Pharmazeutische Zusammensetzungen können pharmazeutisch geeignete Salze, zusätzlich Puffer, Tonizitätskomponenten oder pharmazeutisch geeignete Träger umfassen. Insbesondere inhibitorische Nukleinsäuren, wie antisense Nukleinsäuren, siRNA, shRNA, können in geeigneten therapeutischen Vektorsystemen vorgesehen werden. Pharmazeutische Träger-Substanzen dienen der besseren Verträglichkeit der Zusammensetzung und ermöglichen bessere Löslichkeit sowie bessere Bioverfügbarkeit der Wirksubstanzen. Beispiele hierfür sind Emulgatoren, Verdickungsmittel, Redoxkomponenten, Stärke, Alkohollösungen, Polyethylenglycol oder Lipide. Die Auswahl eines geeigneten pharmazeutischen Trägers hängt stark von der Art der Verabreichung ab. Für orale Verabreichungen können flüssige oder feste Träger verwendet werden, für Injektionen sind flüssige Endzusammensetzungen vorteilhaft.

Vorzugsweise umfasst das erfindungsgemäß zu verwendende Medikament Puffersubstanzen oder tonische Substanzen. Mittels Puffer kann der pH-Wert des Medikaments auf physiologische Konditionen eingestellt werden und weiters können pH-Schwankungen abgeschwächt bzw. gepuffert werden. Ein Beispiel hierfür ist ein Phosphatpuffer. Tonische Substanzen dienen zur Einstellung der Osmolarität und können ionische Substanzen, wie zum Beispiel anorganische Salze, wie NaCl, oder auch nicht-ionische Substanzen, wie zum Beispiel Glycerin oder Kohlenhydrate, umfassen.

Bevorzugterweise ist die erfindungsgemäß zu verwendende Zusammensetzung zur systemischen, topischen, oralen oder intranasalen Verabreichung geeignet hergerichtet. Diese Verabreichungsformen des Medikaments der vorliegenden Erfindung ermöglichen eine schnelle und unkomplizierte Aufnahme. Zur oralen Verabreichung können beispielsweise feste bzw. flüssige Medikamente direkt oder aufgelöst bzw. verdünnt eingenommen werden.

Das erfindungsgemäß zu verwendende Medikament ist vorzugsweise zur intravenösen, intraarteriellen, intramuskulären, intravaskulären, intraperitonealen oder subkutanen Verabreichung geeignet hergerichtet. Hierfür eignen sich beispielsweise Injektionen oder Transfusionen. Verabreichungen direkt in die Blutbahn haben den Vorteil, dass die Wirkstoffe des Medikaments im gesamten Körper verteilt werden und die Zielgewebe schnell erreichen. Weiters sind topische Verabreichungen vorgesehen. Insbesondere eine Verabreichung direkt in oder in die Nähe der Stelle, an der eine Immunantwort ausgelöst oder verstärkt werden soll, z.B. den Ort einer Infektion oder eines Tumors, hat den Vorteil, dass die NK-Zell-Aktivierung vordergründig am Zielort erfolgt.

Neben einer in vivo Verabreichung sind auch ex vivo Behandlungen von NK-Zellen möglich. Hierzu werden NK-Zellen aus einem Patienten isoliert ex vivo erfindungsgemäß behandelt und aktiviert und anschließend wieder in den Patienten zurückgeführt. Ein derartiges ex vivo Verfahren zur Behandlung von Zellen des Immunsystems wird beispielsweise in der WO 2009/073905 (durch Bezugnahme hierin aufgenommen) beschrieben, und kann umgelegt auf NK-Zellen erfindungsgemäß verwendet werden. Vorzugsweise erfolgt die ex vivo Variante des erfindungsgemäßen Verfahrens in Kombination mit den oben genannten zusätzlichen NK-Zell-Aktivatoren wie Immunzell-stimmulierenden Zytokinen, insbesondere IL-2 und/oder IL-12, einem Interferon oder Interferon-Stimulans, einem Antikörper, oder einem TLR- oder PAMP-Rezeptor Liganden, vorzugsweise von TLR-2, TLR-7 oder TLR-8, sowie Kombiantionen hievon. Es ist möglich das entweder die Cbl-b Inhibition oder die zusätzlichen NK-Zell-Aktivatoren ex vivo an die NK-Zellen verabreicht werden und die weitere Aktivierung in vivo erfolgt, z.B. ex vivo Cbl-b Inbibition und in vivo Verabreichung des zusätzlichen NK-Zell-Aktivators, oder umgekehrt. Vorzugsweise können beide Schritte, Cbl-b Inhibition und zusätzliche NK-Zell-Aktivierung, ex vivo vorgenommen werden. Isolierte und aktivierte NK-Zellen können gezielt an den Zielort verabreicht werden. Die NK-Zellen können ex vivo isoliert werden. Zur spezifischen NK-Zell Aktivierung können ex vivo NK-Zellen in einem Zellisolat, z.B. PBMCs, angereichter oder daraus isoliert werden.

Die vorliegende Erfindung wird weiters durch die folgenden Figuren und Beispiele dargestellt, ohne zwingend darauf beschränkt zu sein.

### Figuren:

Fig 1: PBMCs bzw. daraus isolierte Zellfraktionen wurden mittels Cbl-b siRNA inhibiert und dann mit IL-2 und IL-12 stimuliert und die IFN-gamma Produktion nach 24h gemessen.
Fig. 2: PBMCs bzw. daraus isolierte Zellfraktionen wurden mittels Cbl-b siRNA inhibiert, mittels Koinkubation mit der K562 Tumorzelllinie stimuliert und die IFN-gamma Produktion nach 24h gemessen.
Fig. 3: Isolierte NK-Zellen wurden mittels Cbl-b siRNA inhibiert, und mittels K562 oder IL-2 und IL-12 stimuliert und die TNF-alpha Produktion nach 24h gemessen.
Fig. 4: PBMCs wurden mit Cbl-b siRNA inhibiert, und für 4h mit der Tumorzelllinie SKBR3 und wie angegeben zusätzlich unter Zugabe von Herceptin oder Zytokin inkubiert. Die Zytotoxizität wurde dann aufgrund des freigesetzten zellulären LDH-Enzyms bestimmt, welche mittels enzymatischen Assays gemessen wurde.

### Beispiel 1: Sequenzen

Die folgenden siRNA-Sequenzen wurden zur Inhibierung von Cbl-b verwendet:
A.
   Sense Sequenz:
      GUACUGGUCCGUUAGCAAAUU (SEQ ID Nr. 1)
   Antisense Sequenz:
      5'-PUUUGCUAACGGACCAGUACUU (SEQ ID Nr. 2)
B.
   Sense Sequenz:
      GGUCGAAUUUUGGGUAUUAUU (SEQ ID Nr. 3)
   Antisense Sequenz:
      5'-PUAAUACCCAAAAUUCGACCUU (SEQ ID Nr. 4)

### Beispiel 2: Cbl-b Inhibition in NK-Zellen

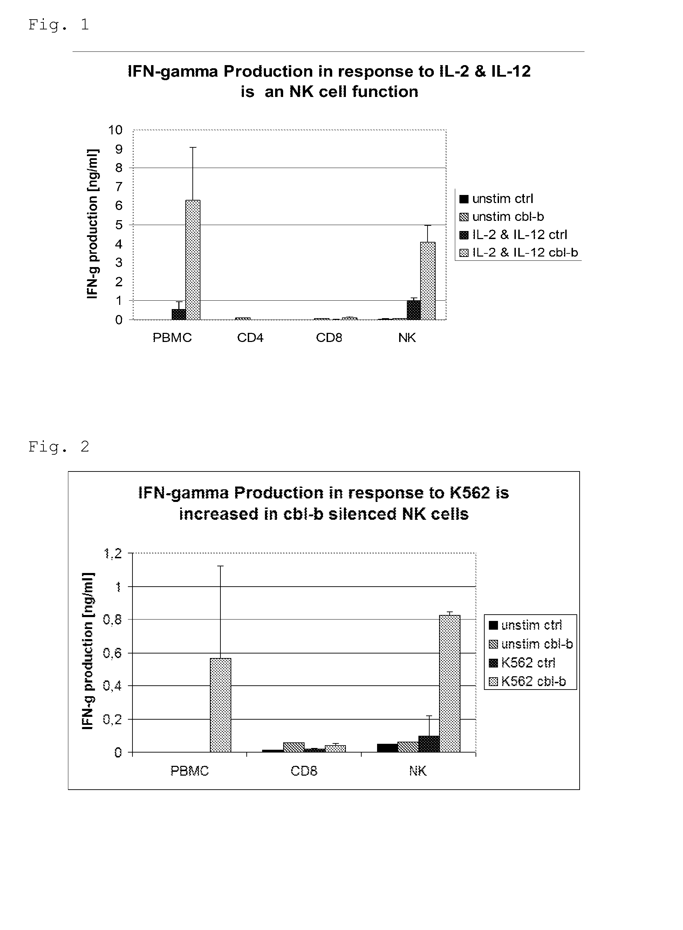

Mittels CPT Röhrchen (Vacutainer) wurde einem Spender Vollblut abgenommen und daraus PBMCs durch Zentrifugation abgetrennt. Aus PBMCs wurden NK-Zellen (inklusive der CD8⁺CD3⁻-Fraktion) und dann die CD8 und CD4 T-Zellen mittels magnetic selection isoliert. Mittels FACS wurde verifiziert, dass die Reinheit der entsprechenden Zellpopulationen zumindest 90% betrug. Sowohl die PBMCs als auch die daraus isolierten Zellfraktionen wurden mit siRNA Cbl-b unter Verwendung eines Amaxa Transfektionsgeräts transfiziert und mit rekombinantem humanem IL-2 (50ng/ml) und IL-12 (10ng/ml) über Nacht in Xvivo15 Medium stimuliert (Fig. 1). Die IFN-gamma Sekretion der so behandelten Zellen wurde dann mittels ELISA gemessen. Das Ergebnis zeigt klar, dass die stark erhöhte IFN-gamma Produktion von Cbl-b-inhibierten PBMCs nach IL-2 und IL-12 Stimulation eindeutig der Reaktion der NK-Zellen zugeordnet werden kann.

### Beispiel 3: Cbl-b Inhibition in NK-Zellen und Kostimmulation

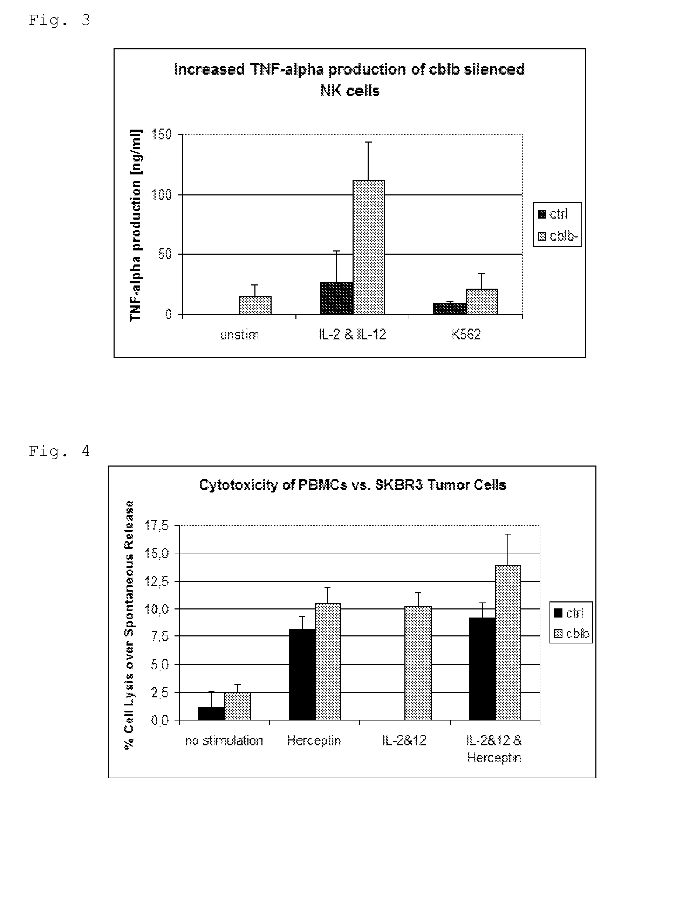

Eine Möglichkeit NK-Zellen zu kostimulieren ist über Tumorzelllinien, deren aberrante Oberflächenmarkerexpression nicht mehr das entsprechende Gleichgewicht zwischen inhibitorischen und aktivatorischen NK-Rezeptoren aufrechterhalten kann und die damit eine Aktivierung der NK-Zellen verursachen, beispielsweise die Tumorzelllinie K562. PBMCs sowie die CD8 und NK-Zellen wurden wie oben beschrieben isoliert und mit Cbl-b siRNA transfiziert. 1x10^5 dieser transfizierten Zellen wurden dann entweder alleine (unstim) oder mit jeweils 6x10^4 K562 Tumorzellen in Xvivo Medium über Nacht inkubiert und die IFNg Sekretion wie oben bestimmt. Inkubation von Cbl-b inhibierten PBMCs mit dieser Tumorzellinie führte ebenfalls wieder zu einer starken IFN-gamma Produktion und wiederum konnte diese IFN-gamma Produktion klar auf den Beitrag der NK-Zellen zurückgeführt werden (Fig. 2). Beide Stimulationsmethoden führten auch zu einem Anstieg der TNF-alpha Produktion von NK-Zellen nach Cbl-b Inhibierung (Fig. 3).

### Beispiel 4: Tumorzytotoxizität durch Cbl-b Inhibition in NK-Zellen

Eine der Hauptfunktionen von NK-Zellen im Kontext der Tumorentstehung ist die direkte Zerstörung von Tumorzellen. Es wurde daher getestet, ob Cbl-b inhibierte PBMCs besser in der Lage sind Tumorzellen zu zerstören. Als Target Zelllinie wurde dabei die Her2-positive SKBR3 Brustkarzinomlinie verwendet, da in diesem Kontext auch der in der Tumortherapie eingesetzte Antikörper gegen Her2 getestet werden kann (Trastuzumab oder Herceptin). PBMCs wurden wieder wie oben beschrieben isoliert und mit Cbl-b siRNA transfiziert und entweder alleine oder mit 4x10^4 SKBR3-Zellen in Xvivo Medium für 4h inkubiert. Zusätzlich wurden in den mit Herceptin bezeichneten Konditionen 10µg/ml Herceptin-Anitkörper zugegeben sowie in den mit IL-2 und IL-12 bezeichneten Konditionen wie oben beschrieben stimuliert. Die Zytotoxizität der PBMCs auf die SKBR3-Tumorzellen wurden dann durch die colometrische Messung der Freisetzung des Enzyms LDH aus dem Tumorzellzytosol bestimmt. Der spontane Release dieses Enzyms aus den PBMCs bzw. den Tumorzellen wurde wie vom Colometrie-Messungskit Hersteller (Biovision) empfohlen aus entsprechenden Einzelkontrollkonditionen bestimmt und abgezogen. Dabei zeigte sich, dass die Zelllyse durch Cbl-b-inhibierte Immunzellen grundsätzlich höher war als durch die mit Kontroll siRNA behandelten Zellen, wobei insbesondere die gleichzeitige Stimulation mit IL-2 und IL-12 zu einer signifikanten Erhöhung der Tumorzelllyse führte (Fig. 4).

Diese in vitro Ergebnisse zeigen daher, dass gleichzeitiges Inhibieren von Cbl-b in Zellen des adaptiven und des angeborenen Immunsystems ex vivo in unseparierten humanen PBMCs möglich ist, und führen weiters zur Schlussfolgerung, dass Cbl-b Inhibierung in NK-Zellen eine rationale Grundlage zur Kombination von Cbl-b Inhibierung mit Tumortherapien wie Gabe von rekombinantem IL-2 oder gegen Tumorantigene gerichteter therapeutischer Antikörper schafft.

## Patentansprüche

1. Cbl-b Inhibitor zur Verwendung in einem Verfahren zur therapeutischen Behandlung eines Patienten umfassend die Einbringung des Cbl-b Inhibitors in NK-Zellen im Patienten, wodurch die NK-Zellen immunaktiviert werden.

2. Verfahren zur Immunaktivierung von NK-Zellen umfassend den Schritt der Reduzierung oder Inhibierung der Cbl-b Funktion in den NK-Zellen, insbesondere durch Verabreichung eines Cbl-b Inhibitors.

3. Cbl-b Inhibitor nach Anspruch 1 oder Verfahren nach Anspruch 2 zur Behandlung von Krebs, einer Virusinfektion, einer bakteriellen Infektion, insbesondere einer chronischen Infektion mit intrazellulär persistenten Bakterien, oder einer Parasiteninfektion, insbesondere von intrazellulären Parasiten oder einer Mykose, in dem Patienten.

4. Cbl-b Inhibitor oder Verfahren nach einem der Ansprüche 1 bis 3, **dadurch gekennzeichnet, dass** der Cbl-b Inhibitor zusammen mit einer weiteren NK-Zell stimulierenden Substanz verabreicht wird, insbesondere mit einer Substanz, welche IFN-gamma-Produktion in NK-Zellen bewirkt.

5. Cbl-b Inhibitor oder Verfahren nach einem der Ansprüche 1 bis 4, **dadurch gekennzeichnet, dass** der Cbl-b Inhibitor zusammen mit einem Immunzell-stimmulierenden Zytokin, insbesondere ein Zytokin der common-gamma-chain Zytokine, ein Zytokin des adaptiven als auch des angeborenen Immunsystems oder ein Effektor-Zellzytokin, einem Interferon oder Interferon-Stimulans, einem Antikörper mit einer Fc-Domäne, oder einem TLR- oder PAMP-Rezeptor Liganden, insbesondere TLR- oder PAMP-Rezeptor Agonisten, sowie Kombinationen hiervon verabreicht wird.

6. Cbl-b Inhibitor oder Verfahren nach Anspruch 5, **dadurch gekennzeichnet, dass** der Cbl-b Inhibitor zusammen mit mindestens einem aus IL-2, IL-15, IL-21, IL-12, IL-23, IL-27, IL-1, IL-17, IL-18, Imiquimod, Resiquimod, ssPolyU-Nukleotide, Loxoribine, Gardiquimod, CL075, CL097, CL264, 3M002, Interferon-alpha, einem Interferon-alpha-Stimulans, poly(I:C) Oligonukleotide, CpG Oligonukleotide, CD205-Liganden oder CD206-Liganden, sowie Kombinationen hiervon, verabreicht wird.

7. Cbl-b Inhibitor oder Verfahren nach einem der Ansprüche 1 bis 6, **dadurch gekennzeichnet, dass** der Cbl-b Inhibitor ausgewählt ist aus inhibierenden Nukleinsäuren, insbesondere antisense Oligonukleotiden, siRNA oder shRNA.

8. Cbl-b Inhibitor oder Verfahren nach einem der Ansprüche 1 bis 6, **dadurch gekennzeichnet, dass** der Cbl-b Inhibitor spezifisch die enzymatische E3-Ligase-Aktivität, die intrazelluläre Assoziation von Cbl-b mit seinen Interaktionspartner inhibiert oder die die Expression von Cbl-b inhibiert.

9. Cbl-b Inhibitor oder Verfahren nach einem der Ansprüche 1 bis 8, **dadurch gekennzeichnet, dass** der Cbl-b Inhibitor an eineinen Liganden eines NK-Zell-Erkennungsmoleküls gekoppelt ist und dadurch gezielt in NK-Zellen wirksam wird.

10. Cbl-b Inhibitor oder Verfahren nach einem der Ansprüche 1 bis 9, **dadurch gekennzeichnet, dass** der Patient ausgewählt ist aus einem Säugetier oder einem Menschen.

11. Pharmazeutische Zusammensetzung umfassend einen Cbl-b Inhibitor, vorzugsweise wie weiters in einem der Ansprüche 7 bis 9 definiert, und einen zusätzlichen NK-Zell Aktivator ausgewählt aus der Gruppe wie in einem der Ansprüche 4 bis 6 definiert.

12. Zusammensetzung nach Anspruch 11 umfassend einen pharmazeutisch akzeptablen Träger, vorzugsweise geeignet zur intrazellulären Verabreichung in einem Patienten, insbesondere Liposomen oder Mikrosomen Formulierungen.
